(19) Europäisches Patentamt / European Patent Office / Office européen des brevets

(11) **EP 1 433 470 B1**

(12) **EUROPÄISCHE PATENTSCHRIFT**

(45) Veröffentlichungstag und Bekanntmachung des Hinweises auf die Patenterteilung:
**26.03.2008 Patentblatt 2008/13**

(51) Int Cl.:
***A61K 8/41*** *(2006.01)* ***A61K 8/49*** *(2006.01)*
***A61K 8/67*** *(2006.01)* ***A61Q 5/10*** *(2006.01)*

(21) Anmeldenummer: **03029369.0**

(22) Anmeldetag: **19.12.2003**

(54) **Mittel zur Färbung keratinischer Fasern**

Composition for dyeing keratinous fibres

Composition de teinture des fibres kératiniques

(84) Benannte Vertragsstaaten:
**AT BE BG CH CY CZ DE DK EE ES FI FR GB GR HU IE IT LI LU MC NL PT RO SE SI SK TR**

(30) Priorität: **23.12.2002 DE 10260835**

(43) Veröffentlichungstag der Anmeldung:
**30.06.2004 Patentblatt 2004/27**

(73) Patentinhaber: **Henkel Kommanditgesellschaft auf Aktien**
**40589 Düsseldorf (DE)**

(72) Erfinder: **Kleen, Astrid, Dr.**
**40699 Erkrath (DE)**

(56) Entgegenhaltungen:
**WO-A-01/97756** **WO-A-02/096382**
**DE-A- 4 137 971**

EP 1 433 470 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung betrifft Mittel zur Färbung keratinischer Fasern, die ein Indolinderivat und ein p-Phenylendiaminderivat in Kombination mit einem speziellen Pflegestoff enthalten, sowie ein Verfahren zur Färbung von Haaren mit diesen Mitteln.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so dass dann sehr viel schneller eine vielfach unerwünschte Nuancenverschiebung oder gar eine sichtbare "Entfärbung" eintritt.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus. Für natürlich wirkende Färbungen muss üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet. Dennoch können auch diese Färbemittel hinsichtlich der Nuancenstabilität und Dauerhaftigkeit noch nicht alle Wünsche befriedigen. In regelmäßigen Abständen ist es daher erforderlich nicht nur die frisch nachgewachsenen Ansätze sondern auch die Haarlängen erneut zu behandeln, um eine einheitliche Färbung zu ermöglichen.

Schließlich hat in jüngster Zeit ein neuartiges Färbeverfahren große Beachtung gefunden. Bei diesem Verfahren werden Vorstufen des natürlichen Haarfarbstoffes Melanin auf das Haar aufbracht; diese bilden dann im Rahmen oxidativer Prozesse im Haar naturanaloge Farbstoffe aus. Ein solches Verfahren mit 5,6-Dihydroxyindolin als Farbstoffvorprodukt wurde in der EP-B1-530 229 beschrieben. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so dass auf keine weiteren Oxidationsmittel zurückgegriffen werden muss. Bei Personen mit ursprünglich mittelblondem bis braunem Haar kann das Indolin als alleinige Farbstoffvorstufe eingesetzt werden. Mithilfe dieses Färbeverfahrens sind jedoch lediglich Naturnuancen zugänglich.

Im Rahmen der WO02/096382 sowie der DE-41 37 971 werden oxidative Färbemittel offenbart, die eine Kombination aus einem p-Phenylendiaminderivat mit 5,6-Duihydroxyindolin enthalten.

Im Rahmen der WO01/97756 werden oxidative Färbemittel offenbart, die eine Kombination aus einem p-Phenylendiaminderivat und Vitamin B6 enthalten.

Es bestand daher nach wie vor die Aufgabe, Färbemittel zu entwickeln, die eine stabile und langanhaltende Färbung ermöglichen.

Überraschenderweise wurde nun gefunden, dass Färbemittel, die eine spezielle Farbstoffkombination sowie einen sorgfältig ausgewählten Pflegestoff enthalten, langanhaltende oxidative Färbungen ermöglichen.

Ein erster Gegenstand der vorliegenden Erfindung sind daher Mittel zur Färbung keratinischer Fasern, insbesondere menschlicher Haare, enthaltend in einem kosmetisch akzeptablen Träger

(a) mindestens ein Indolinderivat,
(b) mindestens ein p-Phenylendiaminderivat und
(c) mindestens einen Pflegestoff, ausgewählt aus

(c2) Ectoin oder Ectoinderivaten,

Unter keratinischen Fasern sind dabei erfindungsgemäß Pelze, Wolle, Federn und insbesondere menschliche Haare zu verstehen. Obwohl die erfindungsgemäßen Oxidationsfärbemittel in erster Linie zum Färben von Keratinfasern geeignet sind, steht prinzipiell einer Verwendung auch auf anderen Gebieten, insbesondere in der Farbphotographie, nichts entgegen.

Mithilfe der erfindungsgemäßen Wirkstoffkombination kann einer oder auch mehrere der folgenden Vorteile erreicht werden. So ist es beispielsweise möglich,

- den Gehalt an Farbstoffvorprodukten in den Färbemitteln zu reduzieren,
- langanhaltende Färbungen zu erhalten, so dass ein Nachfärben der Haarlängen seltener bis gar nicht erforderlich wird,
- die Kopfhaut weniger zu irritieren und/oder
- die Fasern weniger zu strapazieren als dies bei herkömmlichen Färbemitteln der Fall ist.

Da es sich bei den erfindungsgemäß eingesetzten Farbstoffvorprodukten um AminoVerbindungen handelt, lassen sich aus diesen in üblicher Weise die bekannten Säureadditionssalze herstellen. Alle Aussagen dieser Schrift und demgemäss der beanspruchte Schutzbereich beziehen sich daher sowohl auf die in freier Form vorliegenden Verbindungen als auch auf deren wasserlösliche, physiologisch verträglichen Salze. Beispiele für solche Salze sind die Hydrochloride, die Hydrobromide, die Sulfate, die Phosphate, die Acetate, die Propionate, die Citrate und die Lactate. Die Hydrochloride und die Sulfate sind dabei besonders bevorzugt.

Erfindungsgemäß ist es bevorzugt, wenn die Färbemittel als Komponente (a) ein Indolinderivat der Formel (1) oder eines seiner physiologisch verträglichen Salze mit einer organischen oder anorganischen Säure

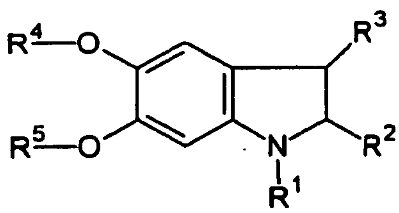

**(I)**

enthalten, wobei unabhängig voneinander

- $R^1$ steht für Wasserstoff, eine $C_1$-$C_4$-Alkylgruppe oder eine $C_1$-$C_4$-Hydroxyalkylgruppe,
- $R^2$ steht für Wasserstoff oder eine -COOH-Gruppe, wobei die -COOH-Gruppe auch als Salz mit einem physiologisch verträglichen Kation vorliegen kann,
- $R^3$ steht für Wasserstoff oder eine $C_1$-$C_4$-Alkylgruppe,
- $R^4$ steht für Wasserstoff, eine $C_1$-$C_4$-Alkylgruppe oder eine Gruppe -CO-$R^6$, in der $R^6$ steht für eine $C_1$-$C_4$-Alkylgruppe, und
- $R^5$ steht für eine der unter $R^4$ genannten Gruppen.

Beispiele für die als Substituenten in den erfindungsgemäßen Verbindungen genannten $C_1$- bis $C_4$-Alkylgruppen sind die Gruppen Methyl, Ethyl, Propyl, Isopropyl und Butyl. Ethyl und Methyl sind bevorzugte Alkylgruppen. Weiterhin können als bevorzugte Beispiele für eine $C_1$- bis $C_4$-Monohydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt.

Besonders bevorzugte Indolinderivat der Formel (I) sind ausgewählt aus 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin und 5,6-Dihydroxyindolin-2-carbonsäure sowie deren physiologisch verträglichen Salzen.

Als ganz besonders bevorzugtes Indolinderivat gilt erfindungsgemäß 5,6-Dihydroxyindolin oder eines seiner physiologisch verträglichen Salze.

Die Indolin-Derivate sind in den erfindungsgemäßen Mitteln bevorzugt in Mengen von 0,05-10 Gew.-%, vorzugsweise 0,2-5 Gew.-% enthalten.

Weiterhin ist es erfindungsgemäß bevorzugt, wenn die Komponente (b) ein p-Phenylendiaminderivat der Formel (II) oder eines seiner physiologisch verträglichen Salze

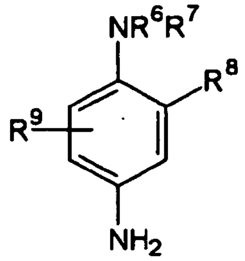

(II)

ist, wobei

- $R^6$ steht für ein Wasserstoffatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$- bis $C_4$)-Alkoxy-($C_1$- bis $C_4$)-alkylrest, einen 4'-Aminophenylrest oder einen $C_1$- bis $C_4$-Alkylrest, der mit einer stickstoffhaltigen Gruppe, einem Phenyl- oder einem 4'-Aminophenylrest substituiert ist;
- $R^7$ steht für ein Wasserstoffatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$- bis $C_4$)-Alkoxy-($C_1$- bis $C_4$)-alkylrest oder einen $C_1$- bis $C_4$-Alkylrest, der mit einer stickstoffhaltigen Gruppe substituiert ist;
- $R^8$ steht für ein Wasserstoffatom, ein Halogenatom, wie ein Chlor-, Brom-, Iod- oder Fluoratom, einen $C_1$- bis $C_4$-Alkylrest, einen $C_1$- bis $C_4$-Monohydroxyalkylrest, einen $C_2$-bis $C_4$-Polyhydroxyalkylrest, einen $C_1$- bis $C_4$-Hydroxyalkoxyrest, einen $C_1$- bis $C_4$-Acetylaminoalkoxyrest, einen $C_1$- bis $C_4$- Mesylaminoalkoxyrest oder einen $C_1$- bis $C_4$-Carbamoylaminoalkoxyrest;
- $R^9$ steht für ein Wasserstoffatom, ein Halogenatom oder einen $C_1$- bis $C_4$-Alkylrest oder
- wenn $R^8$ und $R^9$ in ortho-Stellung zueinander stehen, können sie gemeinsam eine verbrückende $\alpha,\omega$-Alkylendioxogruppe, wie beispielsweise eine Ethylendioxygruppe bilden.

Beispiele für die als Substituenten in den erfindungsgemäßen Verbindungen genannten $C_1$- bis $C_4$-Alkylreste sind die Gruppen Methyl, Ethyl, Propyl, Isopropyl und Butyl. Ethyl und Methyl sind bevorzugte Alkylreste. Erfindungsgemäß bevorzugte $C_1$- bis $C_4$-Alkoxyreste sind beispielsweise eine Methoxy- oder eine Ethoxygruppe. Weiterhin können als bevorzugte Beispiele für eine $C_1$- bis $C_4$-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte $C_2$- bis $C_4$-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, $C_1$- bis $C_4$-Monoalkylaminogruppen, $C_1$- bis $C_4$-Dialkylaminogruppen, $C_1$- bis $C_4$-Trialkylammoniumgruppen, $C_1$- bis $C_4$-Monohydroxyalkylaminogruppen, Imidazolinium und Ammonium.

Erfindungsgemäß sind die p-Phenylendiaminderivat der Formel (II) bevorzugt, die ausgewählt sind aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Besonders bevorzugte p-Phenylendiaminderivate der Formel (II) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

2-(β-Hydroxyethyl)-p-phenylendiamin oder eines seiner physiologisch verträglichen Salze ist ein ganz besonders bevorzugtes p-Phenylendiaminderivat der Formel (II).

Die erfindungsgemäßen Färbemittel können ferner als Komponente (c2) Ectoin oder Ectoinderivate, enthalten.

Erfindungsgemäß werden unter dem Begriff "Ectoin und Ectoinderivate" Verbindungen der Formel (III)

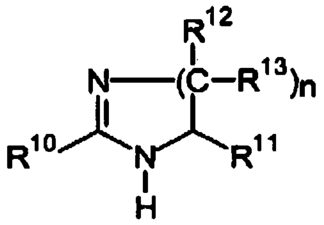 (IIIa) 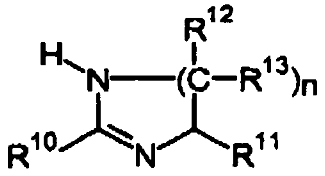 (IIIb)

und/oder deren physiologisch verträglichen Salzes und/oder einer isomeren oder stereoisomeren Form verstanden, wobei

$R^{10}$ steht für ein Wasserstoffatom, einen verzweigten oder unverzweigten $C_1$ - $C_4$-Alkylrest oder einen $C_2$ - $C_4$-Hydroxyalkylrest,

$R^{11}$ steht für ein Wasserstoffatom, eine Gruppierung $-COOR^{14}$ oder eine Gruppierung - $CO(NH)R^{14}$, wobei $R^{14}$ für ein Wasserstoffatom, einen $C_1$ - $C_4$-Alkylrest, einen Aminosäurerest, einen Dipeptid- oder einen Tripeptidrest stehen kann,

$R^{12}$ und $R^{13}$ stehen unabhängig voneinander für ein Wasserstoffatom, einen $C_1$ - $C_4$-Alkylrest oder einer der beiden Reste steht für eine Hydroxygruppe und

n steht für eine ganze Zahl von 1 bis 3.

Geeignete physiologisch verträgliche Salze der allgemeinen Verbindungen gemäß der Formel (IIIa) oder (IIIb) sind beispielsweise die Alkali-, Erdalkali-, Ammonium-, Triethylamin- oder Tris-(2-hydroxyethyl)aminsalze sowie solche, die sich aus der Umsetzung von Verbindungen gemäß der Formel (IIIa) oder (IIIb) mit anorganischen und organischen Säuren wie Salzsäure, Phosphorsäure, Schwefelsäure, verzweigten oder unverzweigten, substituierten oder unsubstituierten (beispielsweise durch eine oder mehrere Hydroxygruppen) $C_1$ - $C_4$- Mono- oder Dicarbonsäuren, aromatische Carbonsäuren und Sulfonsäuren wie Essigsäure, Citronensäure, Benzoesäure, Maleinsäure, Fumarsäure, Weinsäure und p-Toluolsulfonsäure ergeben. Beispiele für besonders bevorzugte physiologisch verträgliche Salze sind die Na-, K-, Mg- und Ca- und Ammoniumsalze der Verbindungen gemäß der Formel (IIIa) oder (IIIb), sowie die Salze, die sich durch Umsetzung von Verbindungen gemäß der Formel (IIIa) oder (IIIb) mit Salzsäure, Essigsäure, Citronensäure und Benzoesäure ergeben.

Unter isomeren oder stereoisomeren Formen der Verbindungen gemäß Formel (IIIa) oder (IIIb) werden erfindungsgemäß alle auftretenden optischen Isomere, Diastereomere, Racemate, Zwitterionen, Kationen oder Gemische davon verstanden.

Unter dem Begriff Aminosäure werden die stereoisomeren Formen, z.B. D- und L-Formen, folgender Verbindungen verstanden:

> Asparagin, Arginin, Asparaginsäure, Glutamin, Glutaminsäure, β-Alanin, γ-Aminobutyrat, $N_\varepsilon$-Acetyllysin, $N_\delta$-Acetylornitin, $N_\gamma$-Acetyldiaminobutyrat, $N_\alpha$-Acetytdiaminobutyrat, Histidin, Isoleucin, Leucin, Methionin, Phenylalanin, Serin, Threonin und Tyrosin.
>
> L-Aminosäuren sind bevorzugt. Aminosäurereste leiten sich von den entsprechenden Aminosäuren ab. Die folgenden Aminosäurereste sind bevorzugt:
>
> Gly, Ala, Ser, Thr, Val, β-Ala, γ-Aminobutyrat, Asp, Glu, Asn, Aln, $N_\varepsilon$-Acetyllysin, $N_\delta$-Acetylornithin, $N_\gamma$-Acetyldiaminobutyrat, $N_\alpha$-Acetyldiaminobutyrat.

Die Kurzschreibweise der Aminosäuren erfolgte nach der allgemein üblichen Schreibweise. Die Di- oder Tripeptidreste sind in ihrer chemischen Natur nach Säureamide und zerfallen bei der Hydrolyse in 2 oder 3 Aminosäuren. Die Aminosäuren in dem Di- oder Tripeptidrest sind durch Amidbindungen miteinander verbunden.

Bezüglich der Herstellung der Di- und Tripeptidreste wird ausdrücklich auf die EP 0 671 161 A1 der Firma Marbert verwiesen. Auch Beispiele für Di- und Tripeptidreste sind der Offenbarung der EP 0 671 161 A1 zu entnehmen.

Beispiele für $C_1$ - $C_4$-Alkylgruppen in den erfindungsgemäßen Verbindungen sind Methyl, Ethyl, n-Propyl, Isopropyl, n-Butyl, Isobutyl und tert.-Butyl. Bevorzugte Alkylgruppen sind Methyl und Ethyl, Methyl ist eine besonders bevorzugte Alkylgruppe. Bevorzugte $C_2$ - $C_4$-Hydroxyalkylgruppen sind die Gruppen 2-Hydroxyethyl, 3-Hydroxypropyl oder 4-Hydroxybutyl; 2-Hydroxyethyl ist eine besonders bevorzugte Hydroxyalkylgruppe.

Es kann erfindungsgemäß weiterhin bevorzugt sein, ein Färbemittel zu formulieren, das die Komponenten (a), (b), (c1) und (c2) enthält.

Die erfindungsgemäßen Färbemittel können weiterhin als Komponente (c1) Vitamine, Pro-Vitamine und Vitaminvorstufen der Gruppen A, B, E und H enthalten.

Zur Gruppe der als Vitamin A bezeichneten Substanzen gehören das Retinol (Vitamin $A_1$) sowie das 3,4-Didehydroretinol (Vitamin $A_2$). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie

dessen Ester wie das Palmitat und das Acetat in Betracht. Die erfindungsgemäß verwendeten Zubereltungen enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.

- Vitamin $B_1$ (Thiamin)
- Vitamin $B_2$ (Riboflavin)
- Vitamin $B_3$. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendetenen Mitteln bevorzugt in Mengen von 0,05 bis 1 Gew.-%, bezogen auf das gesamte Mittel, enthalten ist.
- Vitamin $B_5$ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin $B_5$-Typs sind in den erfindungsgemäß verwendeten Mitteln bevorzugt in Mengen von 0,001 - 5 Gew.-%, bezogen auf das gesamte Mittel, enthalten. Mengen von 0,01 - 1 Gew.-% sind besonders bevorzugt.
- Vitamin $B_6$ (Pyridoxin sowie Pyridoxamin und Pyridoxal).

Unter Vitamin E werden erfindungsgemäß die Tocopherale, insbesondere $\alpha$-Tocopherol verstanden. Tocopherol und seine Derivate, worunter insbesondere die Ester wie das Acetat, das Nicotinat, das Phosphat und das Succinat fallen, sind in den erfindungsgemäß verwendeten Mitteln bevorzugt in Mengen von 0,05-1 Gew.-%, bezogen auf das gesamte Mittel, enthalten.

Als Vitamin H wird die Verbindung (3a*S*,4*S*, 6a*R*)-2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäß verwendeten Mitteln bevorzugt in Mengen von 0,0001 bis 1,0 Gew.-%, insbesondere in Mengen von 0,001 bis 0,1 Gew.-% enthalten.

Panthenol, Pantolacton, Pyridoxin und seine Derivate sowie Nicotinsäureamid und Biotin sind besonders bevorzugte Komponenten (c1).

Neben den Komponenten (a), (b) und (c) können die erfindungsgemäßen Färbemittel ferner mindestens eine weitere Entwicklerkomponente enthalten.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt.

Es kann erfindungsgemäß bevorzugt sein, als weitere Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E1) entsprechen, sowie ihre physiologisch verträglichen Salze:

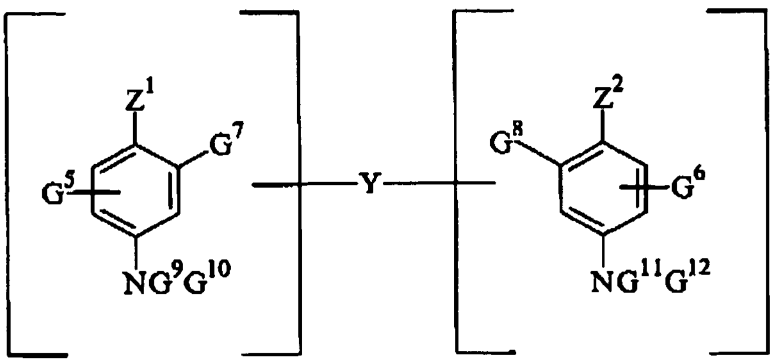

(E1)

wobei:

- $Z^1$ und $Z^2$ stehen unabhängig voneinander für einen Hydroxyl- oder $NH_2$-Rest, der gegebenenfalls durch einen $C_1$- bis $C_4$-Alkylrest, durch einen $C_1$- bis $C_4$-Hydroxyalkylrest und/oder durch eine Verbrückung Y substituiert ist oder der gegebenenfalls Teil eines verbrückenden Ringsystems ist,

- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/ oder einem oder mehreren Heteroatomen wie Sauerstoff-, Schwefel- oder Stickstoffatomen unterbrochen oder beendet sein kann und eventuell durch einen oder mehrere Hydroxyl- oder $C_1$- bis $C_8$-Alkoxyreste substituiert sein kann, oder eine direkte Bindung,
- $G^5$ und $G^6$ stehen unabhängig voneinander für ein Wasserstoff- oder Halogenatom, einen $C_1$- bis $C_4$-Alkylrest, einen $C_1$- bis $C_4$-Monohydroxyalkylrest, einen $C_2$- bis $C_4$- Polyhydroxyalkylrest, einen $C_1$- bis $C_4$-Aminoalkylrest oder eine direkte Verbindung zur Verbrückung Y,
- $G^7$, $G^8$, $G^9$, $G^{10}$, $G^{11}$ und $G^{12}$ stehen unabhängig voneinander für ein Wasserstoffatom, eine direkte Bindung zur Verbrückung Y oder einen $C_1$- bis $C_4$-Alkylrest,
  mit den Maßgaben, dass
- die Verbindungen der Formel (E1) nur eine Verbrückung Y pro Molekül enthalten und
- die Verbindungen der Formel (E1) mindestens eine Aminogruppe enthalten, die mindestens ein Wasserstoffatom trägt.

Die in Formel (E1) verwendeten Substituenten sind erfindungsgemäß analog zu den obigen Ausführungen definiert.

Bevorzugte zweikernige Entwicklerkomponenten der Formel (E1) sind insbesondere: N,N'-Bis-(β-hydroxyethyl)-N,N'-bis-(4'-aminophenyl)-1,3-diamino-propan-2-ol, N, N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N, N'-bis-(4'-amino-3'-methylphenyl)-ethylendiamin, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Besonders bevorzugte zweikernige Entwicklerkomponenten der Formel (E1) sind N,N'-Bis-(β-hydroxyethyl)-N, N'-bis-(4'-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Bis-(2-hydroxy-5-aminophenyl)-methan ist eine ganz besonders bevorzugte zweikernige Entwicklerkomponente der Formel (E1).

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E2)

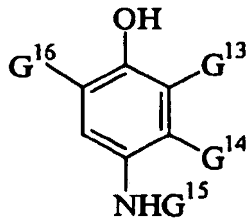

(E2)

wobei:

- $G^{13}$ steht für ein Wasserstoffatom, ein Halogenatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$- bis $C_4$)-Alkoxy-($C_1$- bis $C_4$)-alkylrest, einen $C_1$- bis $C_4$-Aminoalkylrest, einen Hydroxy-($C_1$- bis $C_4$)-alkylaminorest, einen $C_1$- bis $C_4$-Hydroxyalkoxyrest, einen $C_1$- bis $C_4$-Hydroxyalkyl-($C_1$-bis $C_4$)-aminoalkylrest oder einen (Di-$C_1$- bis $C_4$-Alkylamino)-($C_1$- bis $C_4$)-alkylrest, und
- $G^{14}$ steht für ein Wasserstoff- oder Halogenatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$- bis $C_4$)-Alkoxy-($C_1$- bis $C_4$)-alkylrest, einen $C_1$- bis $C_4$-Aminoalkylrest oder einen $C_1$- bis $C_4$-Cyanoalkylrest,
- $G^{15}$ steht für Wasserstoff, einen $C_1$- bis $C_4$-Alkylrest, einen $C_1$- bis $C_4$-Monohydroxyalkylrest, einen $C_2$- bis $C_4$-Polyhydroxyalkylrest, einen Phenylrest oder einen Benzylrest, und
- $G^{16}$ steht für Wasserstoff oder ein Halogenatom.

Die in Formel (E2) verwendeten Substituenten sind erfindungsgemäß analog zu den obigen Ausführungen definiert.

Bevorzugte p-Aminophenole der Formel (E2) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol; 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethylaminomethyl)-phenol sowie ihre physiologisch verträglichen Salze.

Ganz besonders bevorzugte Verbindungen der Formel (E2) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4'-Methoxyphenyl)-amino-3-amino-pyridin, 2,3-Diamino-6-methoxy-pyridin, 2-(β-Methoxyethyl)-amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)-amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)-amino-1-methylpyrazol.

Bevorzugte Pyrazol-Pyrimidin-Derivate sind insbesondere die Derivate des Pyrazol-[1,5-a]-pyrimidin der folgenden Formel (E3) und dessen tautomeren Formen, sofern ein tautomeres Gleichgewicht besteht:

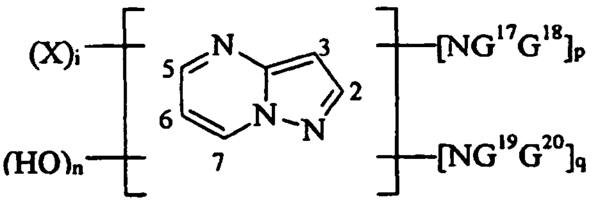

(E3)

wobei:

- $G^{17}$, $G^{18}$, $G^{19}$ und $G^{20}$ unabhängig voneinander stehen für ein Wasserstoffatom, einen $C_1$- bis $C_4$-Alkylrest, einen Aryl-Rest, einen $C_1$- bis $C_4$-Hydroxyalkylrest, einen $C_2$- bis $C_4$-Polyhydroxyalkylrest einen ($C_1$- bis $C_4$)-Alkoxy-($C_1$- bis $C_4$)-alkylrest, einen $C_1$- bis $C_4$-Aminoalkylrest, der gegebenenfalls durch ein Acetyl-Ureid- oder einen Sulfonyl-Rest geschützt sein kann, einen ($C_1$- bis $C_4$)-Alkylamino-($C_1$- bis $C_4$)-alkylrest, einen Di-[($C_1$- bis $C_4$)-alkyl]-($C_1$- bis $C_4$)-aminoalkylrest, wobei die Dialkyl-Reste gegebenenfalls einen Kohlenstoffzyklus oder einen Heterozyklus mit 5 oder 6 Kettengliedern bilden, einen $C_1$- bis $C_4$-Hydroxyalkyl- oder einen Di-($C_1$- bis $C_4$)-[Hydroxyalkyl]-($C_1$- bis $C_4$)-aminoalkylrest,
- die X-Reste stehen unabhängig voneinander für ein Wasserstoffatom, einen $C_1$- bis $C_4$-Alkylrest, einen Aryl-Rest, einen $C_1$- bis $C_4$-Hydroxyalkylrest, einen $C_2$- bis $C_4$-Polyhydroxyalkylrest, einen $C_1$- bis $C_4$-Aminoalkylrest, einen ($C_1$- bis $C_4$)-Alkylamino-($C_1$- bis $C_4$)-alkylrest, einen Di-[($C_1$- bis $C_4$)alkyl]- ($C_1$- bis $C_4$)-aminoalkylrest, wobei die Dialkyl-Reste gegebenenfalls einen Kohlenstoffzyklus oder einen Heterozyklus mit 5 oder 6 Kettengliedern bilden, einen $C_1$- bis $C_4$-Hydroxyalkyl- oder einen Di-($C_1$- bis $C_4$-hydroxyalkyl)-aminoalkylrest, einen Aminorest, einen $C_1$- bis $C_4$-Alkyl- oder Di-($C_1$- bis $C_4$-hydroxyalkyl)-aminorest, ein Halogenatom, eine Carboxylsäuregruppe oder eine

Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, dass
- die Summe aus p + q ungleich 0 ist,
- wenn p + q gleich 2 ist, n den Wert 0 hat, und die Gruppen $NG^{17}G^{18}$ und $NG^{19}G^{20}$ belegen die Positionen (2,3); (5,6); (6,7); (3,5) oder (3,7);
- wenn p + q gleich 1 ist, n den Wert 1 hat, und die Gruppen $NG^{17}G^{18}$ (oder $NG^{19}G^{20}$) und die Gruppe OH belegen die Positionen (2,3); (5,6); (6,7); (3,5) oder (3,7);

Die in Formel (E3) verwendeten Substituenten sind erfindungsgemäß analog zu den obigen Ausführungen definiert.

Wenn das Pyrazol-[1,5-a]-pyrimidin der obenstehenden Formel (E3) eine Hydroxygruppe an einer der Positionen 2, 5 oder 7 des Ringsystems enthält, besteht ein tautomeres Gleichgewicht, das zum Beispiel im folgenden Schema dargestellt wird:

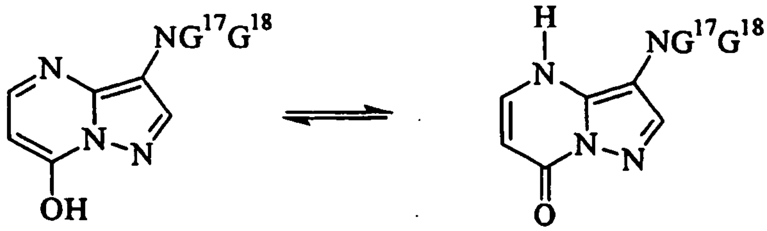

Unter den Pyrazol-[1,5-a]-pyrimidinen der obenstehenden Formel (E3) kann man insbesondere nennen:

- Pyrazol-[1,5-a]-pyrimidin-3,7-diamin;
- 2,5-Dimethyl-pyrazol-[1,5-a]-pyrimidin-3,7-diamin;
- Pyrazol-[1,5-a]-pyrimidin-3,5-diamin;
- 2,7-Dimethyl-pyrazol-[1,5-a]-pyrimidin-3,5-diamin;
- 3-Aminopyrazol-[1,5-a]-pyrimidin-7-ol;
- 3-Aminopyrazol-[1,5-a]-pyrimidin-5-ol;
- 2-(3-Aminopyrazol-[1,5-a]-pyrimidin-7-ylamino)-ethanol;
- 2-(7-Aminopyrazol-[1,5-a]-pyrimidin-3-ylamino)-ethanol;
- 2-[(3-Aminopyrazol-[1,5-a]-pyrimidin-7-yl)-(2-hydroxy-ethyl)-amino]-ethanol;
- 2-[(7-Aminopyrazol-[1,5-a]-pyrimidin-3-yl)-(2-hydroxy-ethyl)-amino]-ethanol;
- 5,6-Dimethylpyrazol-[1,5-a]-pyrimidin-3,7-diamin;
- 2,6-Dimethylpyrazol-[1,5-a]-pyrimidin-3,7-diamin;
- 3-Amino-7-dimethylamino-2,5-dimethylpyrazol-[1,5-a]-pyrimidin;

sowie ihre physiologisch verträglichen Salze und ihre tautomeren Formen, wenn ein tautomers Gleichgewicht vorhanden ist.

Die Pyrazol-[1,5-a]-pyrimidine der obenstehenden Formel (E3) können wie in der Literatur beschrieben durch Zyklisierung ausgehend von einem Aminopyrazol oder von Hydrazin hergestellt werden.

In einer weiteren bevorzugten Ausführungsform enthalten die erfindungsgemäßen Färbemittel mindestens eine weitere Kupplerkomponente.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind

- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Die erfindungsgemäßen Haarfärbemittel enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel. Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1:0,5 bis 1:3, insbesondere 1:1 bis 1:2, enthalten sein können.

Es kann erfindungsgemäß bevorzugt sein, das Indolinderivat in Kombination mit mindestens einer Aminosäure oder einem Oligopeptid einzusetzen. Die Aminosäure ist vorteilhafterweise eine $\alpha$-Aminosäure; ganz besonders bevorzugte $\alpha$-Aminosäuren sind Arginin, Ornithin, Lysin, Serin und Histidin, insbesondere Arginin.

Neben den erfindungsgemäßen Komponenten (a), (b) und (c) können die erfindungsgemäßen Färbemittel in einer weiteren bevorzugten Ausführungsform der vorliegenden Erfindung zur Nuancierung einen oder mehrere direktziehende Farbstoffe enthalten. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-($\beta$-hydroxyethyl)-amino-2-nitrobenzol, 3-Nitro-4-($\beta$-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel einen kationischen direktziehenden Farbstoff enthalten. Besonders bevorzugt sind dabei

(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

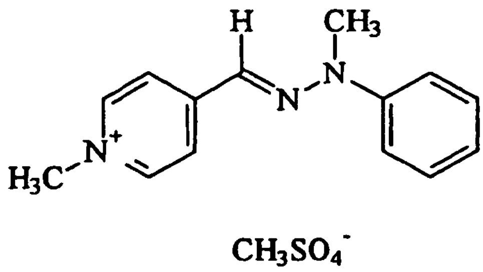

(DZ1)

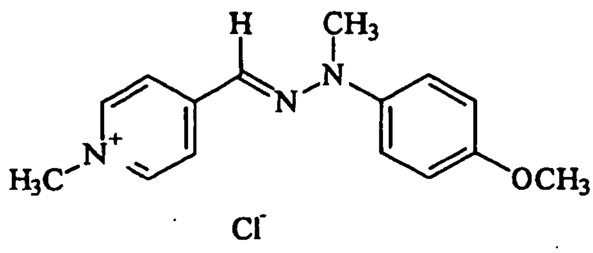

(DZ2)

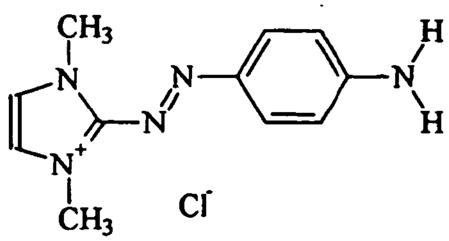

(DZ3)

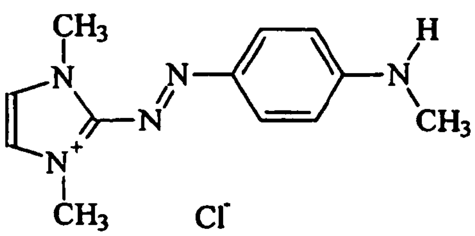

(DZ4)

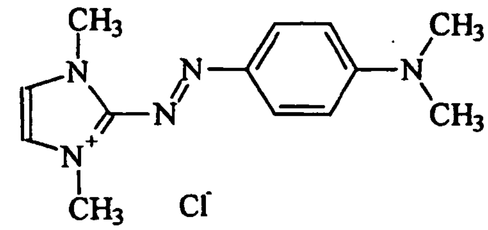

(DZ5)

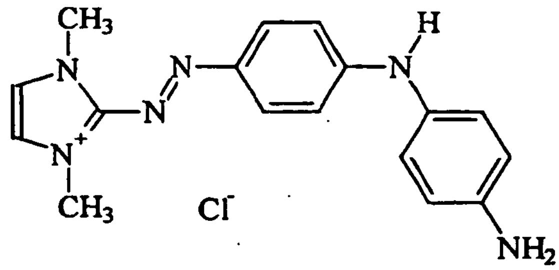

(DZ6)

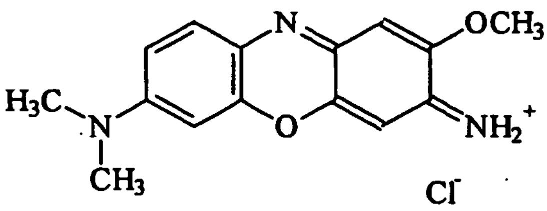

(DZ7)

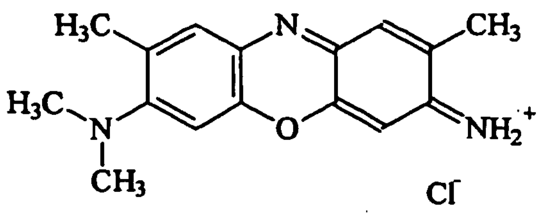

(DZ8)

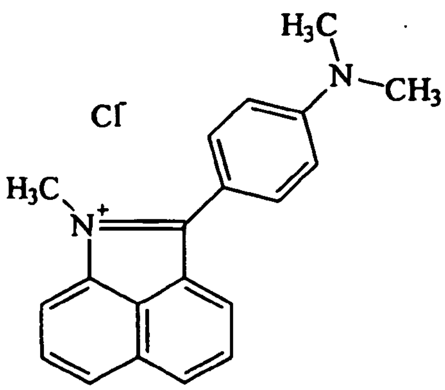

(DZ9)

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Die erfindungsgemäßen Färbemittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,

- lineare Fettsäuren mit 10 bis 22 C-Atomen (Seifen),
- Ethercarbonsäuren der Formel $R-O-(CH_2CH_2O)_x-CH_2-COOH$, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel $R-O(CH_2-CH_2O)_x-SO_3H$, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten $C_8$-$C_{22}$-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- $C_{12}$-$C_{22}$-Fettsäuremono- und -diester von Anlagerungsprodukten von 1 bis 30 Mol Ethylenoxid an Glycerin,
- $C_8$-$C_{22}$-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel $R^1O$-$(Z)_x$. Diese Verbindungen sind durch die folgenden Parameter gekennzeichnet.

Der Alkylrest $R^1$ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest $R^1$ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen $R^1$

- im wesentlichen aus $C_8$- und $C_{10}$-Alkylgruppen,
- im wesentlichen aus $C_{12}$- und $C_{14}$-Alkylgruppen,
- im wesentlichen aus $C_8$- bis $C_{16}$-Alkylgmppen oder
- im wesentlichen aus $C_{12}$- bis $C_{16}$-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine $-COO^{(-)}$- oder $-SO_3^{(-)}$-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethytcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer $C_8$-$C_{18}$-Alkyl- oder Acylgruppe im Molekül mindestens eine freie Aminogruppe und mindestens eine -COOH- oder $-SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das $C_{12-18}$-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCl-Bezeichnungen Quatemium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis (2-Palmitoyloxyethyl)dimethylammoniumchlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyldimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil® - Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat®100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Ferner können die erfindungsgemäßen Färbemittel weitere Wirk-, Hilfs- und Zusatzstoffe, wie beispielsweise

- nichtionische Polymere wie beispielsweise Vinylpyn-olidon/Vinylacrylat-Copolymere, Polyvinylpyrrolidon und VinylpyrrolidonNinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quatemierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z.

B. Bentonit oder vollsynthetische Hydrokolloide wie z.B. Polyvinylalkohol,

- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessemde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe Pyrrolidoncarbonsäuren und deren Salze,
- Vitamine, Provitamine und Vitaminvorstufen der Gruppen C und F,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, $N_2O$, Dimethylether, $CO_2$ und Luft,
- Antioxidantien,

enthalten

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die erfindungsgemäßen Mittel enthalten die Farbstoffvorprodukte bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch denkbar, die Farbstoffvorprodukte in eine pulverförmige oder auch Tabletten-förmige Formulierung zu integrieren.

Unter wässrig-alkoholischen Lösungen sind im Sinne der vorliegenden Erfindung wässrige Lösungen enthaltend 3 bis 70 Gew.-% eines $C_1$-$C_4$-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittels.

Die erfindungsgemäße Komponente (c) kann sowohl gemeinsam mit den Komponenten (a) und (b) konfektioniert sein als auch separat. Falls die Komponente (c) separat konfektioniert ist, kann es erfindungsgemäß bevorzugt sein, die Zubereitung, enthaltend die Komponente (c), unmittelbar vor der Anwendung mit der Zubereitung, enthaltend die Komponenten (a) und (b), zu vermischen. Es kann aber auch erfindungsgemäß bevorzugt sein, die Zubereitung, enthaltend die Komponente (c), unmittelbar vor oder unmittelbar nach der Anwendung einer Zubereitung, enthaltend die Komponenten (a) und (b), einzusetzen.

Die eigentliche oxidative Färbung der Fasern kann grundsätzlich mit Luftsauerstoff erfolgen. Bevorzugt wird jedoch ein chemisches Oxidationsmittel eingesetzt, besonders dann, wenn neben der Färbung ein Aufhelleffekt an

menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, Iodide, Chinone oder bestimmte Enzyme.

Geeignete Metallionen sind beispielsweise $Zn^{2+}$, $Cu^{2+}$, $Fe^{2+}$, $Fe^{3+}$, $Mn^{2+}$, $Mn^{4+}$, $Li^{+}$, $Mg^{2+}$, $Ca^{2+}$ und $Al^{3+}$. Besonders geeignet sind dabei $Zn^{2+}$, $Cu^{2+}$ und $Mn^{2+}$. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.

- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Das eigentliche Haarfärbemittel wird zweckmäßigerweise unmittelbar vor der Anwendung durch Mischung der Zubereitung des Oxidationsmittels mit der Zubereitung, enthaltend die Farbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann die Zubereitung mit den Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Ein zweiter Gegenstand der vorliegenden Erfindung ist die Verwendung der erfindungsgemäßen Wirkstoffkombination zur Färbung keratinischer Fasern.

Ein dritter Gegenstand der vorliegenden Erfindung ist die Verwendung der erfindungsgemäßen Wirkstoffkombination zur Erzielung langanhaltender Färbungen.

Ein vierter Gegenstand der vorliegenden Erfindung ist ein Verfahren zur Färbung keratinischer Fasern, bei dem ein erfindungsgemäßes Haarfärbemittel auf die Fasern aufgetragen wird und nach einer Einwirkzeit wieder abgespült wird.

## Ausführungsbeispiele

Es wurden die in Tabelle I angegebenen Färbecremes A-E der Nuance Hellbraun hergestellt. Die Mengenangaben verstehen sich, sofern nichts anderes vermerkt ist, in Gewichtsprozent. Unmittelbar vor der Anwendung wurden die Färbecremes im Gewichtsverhältnis 1:1 mit einer 6%igen kommerziellen wässrigen Wasserstoffperoxidemulsion vermischt. Anschließend wurden die fertigen Färbemischungen auf Humanhaarsträhnen ("Kerling Naturweiß") mit einem

Flottenverhältnis von 4:1 (Färbemischung : Haar) für 30 min bei Raumtemperatur ausgefärbt. Abschließend wurden die Strähnen gründlich mit Wasser gespült und dann getrocknet.

Tabelle I:

| | Färbecreme A* | Färbecreme B* | Färbecreme C* | Färbecreme D* | Färbecreme E* |
|---|---|---|---|---|---|
| Texapon® NSO[1] | 15 | 15 | 15 | 15 | 15 |
| Dehyton® K[2] | 12 | 12 | 12 | 12 | 12 |
| Lorol® techn.[3] | 4 | 4 | 4 | 4 | 4 |
| Hydrenol® D[4] | 10 | 10 | 10 | 10 | 10 |
| Eumulgin® B1[5] | 0.5 | 0.5 | 0.5 | 0.5 | 0.5 |
| Eumulgin® B2[6] | 0.5 | 0.5 | 0.5 | 0.5 | 0.5 |
| Ascorbinsäure | 0.5 | 0.5 | 0.5 | 0.5 | 0.5 |
| Natriumsulfit | 0.5 | 0.5 | 0.5 | 0.5 | 0.5 |
| Ammoniumdihydrogenphosphat | 1 | 1 | 1 | 1 | 1 |
| Ammoniak | ad pH 10 | ad pH 10 | ad pH 10 | ad pH 10 | ad pH 10 |
| 1-(2-Hydroxyethyl)-2,5-diaminobenzolsulfat | 1.3 | 1.3 | 1.3 | 1.3 | 1.3 |
| 5,6-Dihydroxyindolin-hydrobromid | 0.1 | 0.1 | 0.1 | -- | -- |
| 2-Methylresorcin | 0.09 | 0.09 | 0.09 | 0.09 | 0.9 |
| 4-Chlorresorcin | 0.3 | 0.3 | 0.3 | 0.3 | 0.3 |
| m-Aminophenol | 0.05 | 0.05 | 0.05 | 0.05 | 0.05 |
| 2,7-Dihydroxynaphthalin | 0.23 | 0.23 | 0.23 | 0.23 | 0.23 |
| 2,4,5,6-Tetraaminopyrimidin | 0.03 | 0.03 | 0.03 | 0.03 | 0.03 |
| 1-Methoxy-2-amino-4(2-hydroxyethylamino)benzol-sulfat | 0.03 | 0.03 | 0.03 | 0.03 | 0.03 |
| Vitamin B6 | 0.5 | -- | -- | 0.5 | -- |
| Rona Care® Ectoin[7] | -- | 2.0 | -- | -- | 2.0 |
| Wasser | ad 100 | ad 100 | ad 100 | ad 100 | ad 100 |
| Farbstärke nach Färbung[a] | 100 % | 102 % | 97% | 75 % | 77 % |
| Farbstärke nach 6 Wäschen[b] | 99 % | 98 % | 91 % | 85 % | 85 % |
| Hautvefträglichkeit[c] | + | ++ | 0 | 0 | 0 |
| Haarschonung[d] (Temp.erniedrigung) | + (3.2 °C) | + (3.8 °C) | 0 (5.7 °C) | + (3.5 C) | + (3.9 °C) |

*nicht erfindungsgemäß
[1] Laurylethersulfat, Natriumsalz (ca. 27,5% Aktivsubstanz; INCI-Bezeichnung: Sodium Laureth Sulfate) (Cognis)
[2] N,N-Dimethyl-N-($C_{8-18}$-kokosamidopropyl)ammoniumacetobetain (ca. 30% Aktivsubstanz; INCl-Bezeichnung: Aqua (Water), Cocamidopropyl Betaine) (Cognis)
[3] $C_{12-18}$-Fettalkohol (INCl-Bezeichnung: Coconut alcohol) (Cognis)
[4] $C_{16-18}$-Fettalkohol (INCl-Bezeichnung: Cetearyl alcohol) (Cognis)
[5] Cetylstearylalkohol mit ca. 12 EO-Einheiten (INCl-Bezeichnung: Ceteareth-12) (Cognis)
[6] Cetylstearylalkohol mit ca. 20 EO-Einheiten (INCl-Bezeichnung: Ceteareth-20) (Cognis)
[7] 2-Methyl-1,4,5,6-tetrahydropyrimidin-4-carbonsäure (Merck)

a) Bestimmung der Farbstärke

Nach der Behandlung wurden die erzielte Farbstärke anhand von colorimetrischen Messungen bestimmt. Dazu wurde die Färbung der Strähnen farbmetrisch an 4 Messpunkten mit dem Gerät Datacolor Text Flash, der Firma Data Color International vermessen, die Messergebnisse mit der Software Data Color Tools QC gemäß Formel (I) ausgewertet und in Tabelle 1 zusammengestellt. Als Referenz diente die Färbung einer Strähne, die mit der Färbecreme A gefärbt wurde.

$$\frac{K/S_{Probe}}{K/S_{Referenz}} * 100 = \text{Farbstärke}[\%] \qquad \text{(I)}$$

mit
K = Absorptionskoeffizient
S = Streukoeffizient
K/S = Reflektionskoeffzient

b) Bestimmung der Waschechtheit

Die ausgefärbten Strähnen wurden 6 Handwäschen mit einem marktüblichen Shampoo unterworfen. Nach Trocknung der Strähnen wurde wiederum die Farbstärke bestimmt. Als Referenz diente hier jeweils die Färbung der frisch ausgefärbten Strähne. Die Ergebnisse sind in ebenfalls Tabelle 1 angegeben.

c) Bestimmung der Hautverträglichkeit

Die Hautverträglichkeit wurde im offenen Epikutantest nach Burkhard geprüft, der beispielsweise in Walker et al., Test guidelines for assessment of skin compatibility of cosmetic finished products in man, Food and Chemical Toxicology 34 (1996) 651-660) beschrieben wurde. Es konnte eine bessere Verträglichkeit der erfindungsgemäßen Rezepturen, insbesondere der Rezeptur B, im Vergleich zu den Vergleichsrezepturen C-E festgestellt werden.

d) Bestimmung der Haarschonung

Zur Bestimmung der Haarschonung wurden analoge Ausfärbungen auf Strähnen der Marke "Humanhaar Alkinco 6634" durchgeführt. Die Haarschonung wurde mittels DSC (Thermoanalyse) geprüft. Im Rahmen dieser Methode dient die Schmelzpunktserniedrigung der Fasern als Indikator für die Haarschädigung. Je weniger sich der Schmelzpunkt der gefärbten Haare von dem der unbehandelten Haare unterscheidet, desto haarschonende ist die jeweilige Behandlung. Das ungefärbte Haar zeigt eine Denaturierungstemperatur von 156.8 °C; dieser Messwert diente als Referenz für die Messungen an den gefärbten Strähnen.

## Patentansprüche

1. Mittel zur Färbung keratinischer Fasern, insbesondere menschlicher Haare, enthaltend in einem kosmetisch akzeptablen Träger

   (a) mindestens ein Indolinderivat,
   (b) mindestens ein p-Phenylendiaminderivat und
   (c) mindestens einen Pflegestoff, ausgewählt aus Ectoin oder Ectoinderivaten.

2. Mittel nach Anspruch 1, **dadurch gekennzeichnet, dass** es ein Indolinderivat der Formel (I) oder eines seiner physiologisch verträglichen Salze mit einer organischen oder anorganischen Säure enthält, wobei unabhängig voneinander

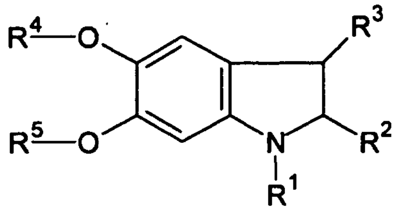

(I)

- $R^1$ steht für Wasserstoff, eine $C_1$-$C_4$-Alkylgruppe oder eine $C_1$-$C_4$-Hydroxyalkylgruppe,
- $R^2$ steht für Wasserstoff oder eine -COOH-Gruppe, wobei die -COOH-Gruppe auch als Salz mit einem physiologisch verträglichen Kation vorliegen kann,
- $R^3$ steht für Wasserstoff oder eine $C_1$-$C_4$-Alkylgruppe,
- $R^4$ steht für Wasserstoff, eine $C_1$-$C_4$-Alkylgruppe oder eine Gruppe -CO-$R^6$, in der $R^6$ steht für eine $C_1$-$C_4$-Alkylgruppe, und
- $R^5$ steht für eine der unter $R^4$ genannten Gruppen.

3. Mittel nach Anspruch 2, **dadurch gekennzeichnet, dass** das Indolinderivat ausgewählt ist aus 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin und 5,6-Dihydroxyindolin-2-carbonsäure sowie deren physiologisch verträglichen Salzen.

4. Mittel nach Anspruch 3, **dadurch gekennzeichnet, dass** das Indolinderivat 5,6-Dihydroxyindolin oder eines seiner physiologisch verträglichen Salze ist.

5. Mittel nach einem der Ansprüche 1 bis 4, **dadurch gekennzeichnet, dass** es mindestens ein p-Phenylendiaminderivat der Formel (II) oder eines seiner physiologisch verträglichen Salze

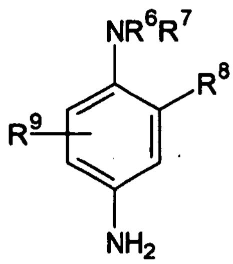

(II)

enthält, wobei

- $R^6$ steht für ein Wasserstoffatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$- bis $C_4$)-Alkoxy-($C_1$- bis $C_4$)-alkylrest, einen 4'-Aminophenylrest oder einen $C_1$- bis $C_4$-Alkylrest, der mit einer stickstoffhaltigen Gruppe, einem Phenyl- oder einem 4'-Aminophenylrest substituiert ist;
- $R^7$ steht für ein Wasserstoffatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$- bis $C_4$)-Alkoxy-($C_1$- bis $C_4$)-alkylrest oder einen $C_1$- bis $C_4$-Alkylrest, der mit einer stickstoffhaltigen Gruppe substituiert ist;
- $R^8$ steht für ein Wasserstoffatom, ein Halogenatom, wie ein Chlor-, Brom-, Iod- oder Fluoratom, einen $C_1$- bis $C_4$-Alkylrest, einen $C_1$- bis $C_4$-Monohydroxyalkylrest, einen $C_2$- bis $C_4$-Polyhydroxyalkylrest, einen $C_1$- bis $C_4$-Hydroxyalkoxyrest, einen $C_1$- bis $C_4$-Acetylaminoalkoxyrest, einen $C_1$- bis $C_4$-Mesylaminoalkoxyrest oder einen $C_1$- bis $C_4$-Carbamoylaminoalkoxyrest;
- $R^9$ steht für ein Wasserstoffatom, ein Halogenatom oder einen $C_1$- bis $C_4$-Alkylrest oder
- wenn $R^8$ und $R^9$ in ortho-Stellung zueinander stehen, können sie gemeinsam eine verbrückende $\alpha,\omega$-Alkylendioxogruppe, wie beispielsweise eine Ethylendioxygruppe bilden.

6. Mittel nach Anspruch 5, **dadurch gekennzeichnet, dass** das p-Phenylendiaminderivat ausgewählt ist aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

**7.** Mittel nach Anspruch 6, **dadurch gekennzeichnet, dass** das p-Phenylendiaminderivat ausgewählt ist aus p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

**8.** Mittel nach Anspruch 7, **dadurch gekennzeichnet, dass** das p-Phenylendiaminderivat 2-(β-Hydroxyethyl)-p-phenylendiamin oder eines seiner physiologisch verträglichen Salze ist.

**9.** Mittel nach einem der Ansprüche 1 bis 8, **dadurch gekennzeichnet, dass** es mindestens eine Kupplerkomponente enthält.

**10.** Mittel nach einem der Ansprüche 1 bis 9, **dadurch gekennzeichnet, dass** es mindestens eine weitere Entwicklerkomponente enthält.

**11.** Mittel nach einem der Ansprüche 1 bis 10, **dadurch gekennzeichnet, dass** es weiterhin mindestens einen direktziehenden Farbstoff enthält.

**12.** Mittel nach Anspruch 11, **dadurch gekennzeichnet, dass** der direktziehende Farbstoff kationisch ist.

**13.** Verfahren zur Färbung keratinischer Fasern, bei dem ein Mittel nach einem der Ansprüche 1 bis 12 auf die Fasern aufgetragen wird und nach einer Einwirkzeit wieder abgespült wird.

## Claims

**1.** Composition for dyeing keratinous fibres, in particular human hair, comprising, in a cosmetically acceptable carrier,

(a) at least one indoline derivative,
(b) at least one p-phenylenediamine derivative and
(c) at least one care substance selected from ectoin or ectoin derivatives.

**2.** Composition according to Claim 1, **characterized in that** it comprises an indoline derivative of the formula (I) or one of its physiologically compatible salts with an organic or inorganic acid, where, independently of one another,

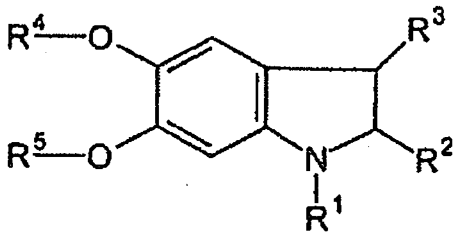

(I)

- $R^1$ is hydrogen, a $C_1$-$C_4$-alkyl group or a $C_1$-$C_4$-hydroxyalkyl group,
- $R^2$ is hydrogen or a -COOH group, where the -COOH group may also be present as salt with a physiologically compatible cation,
- $R^3$ is hydrogen or a $C_1$-$C_4$-alkyl group,
- $R^4$ is hydrogen, a $C_1$-$C_4$-alkyl group or a group
- CO-$R^6$, in which $R^6$ is a $C_1$-$C_4$-alkyl group, and

- $R^5$ is one of the groups specified under $R^4$.

3. Composition according to Claim 2, **characterized in that** the indoline derivative is selected from 5,6-dihydroxyindoline, N-methyl-5,6-dihydroxyindoline, N-ethyl-5,6-dihydroxyindoline, N-propyl-5,6-dihydroxyindoline, N-butyl-5,6-dihydroxyindoline and 5,6-dihydroxyindoline-2-carboxylic acid, and physiologically compatible salts thereof.

4. Composition according to Claim 3, **characterized in that** the indoline derivative is 5,6-dihydroxyindoline or one of its physiologically compatible salts.

5. Composition according to one of Claims 1 to 4, **characterized in that** it comprises at least one p-phenylenediamine derivative of the formula (II) or one of its physiologically compatible salts

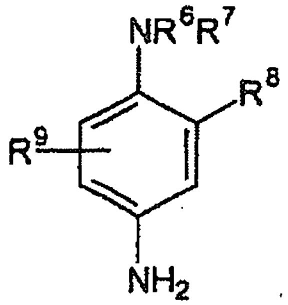

(II)

where

- $R^6$ is a hydrogen atom, a $C_1$-$C_4$-alkyl radical, a $C_1$-$C_4$-monohydroxyalkyl radical, a $C_2$-$C_4$-polyhydroxyalkyl radical, a ($C_1$-$C_4$) -alkoxy- ($C_1$-$C_4$) -alkyl radical, a 4'-aminophenyl radical or a $C_1$-$C_4$-alkyl radical which is substituted by a nitrogen-containing group, a phenyl radical or a 4'-aminophenyl radical;
- $R^7$ is a hydrogen atom, a $C_1$-$C_4$-alkyl radical, a $C_1$-$C_4$-monohydroxyalkyl radical, a $C_2$-$C_4$-polyhydroxyalkyl radical, a ($C_1$-$C_4$) -alkoxy- ($C_1$-$C_4$) -alkyl radical or a $C_1$-$C_4$-alkyl radical which is substituted by a nitrogen-containing group;
- $R^8$ is a hydrogen atom, a halogen atom, such as a chlorine, bromine, iodine or fluorine atom, a $C_1$-$C_4$-alkyl radical, a $C_1$-$C_4$-monohydroxyalkyl radical, a $C_2$-$C_4$-polyhydroxyalkyl radical, a $C_1$-$C_4$-hydroxyalkoxy radical, a $C_1$-$C_4$-acetylaminoalkoxy radical, a $C_1$-$C_4$-mesylaminoalkoxy radical or a $C_1$-$C_4$-carbamoylaminoalkoxy radical;
- $R^9$ is a hydrogen atom, a halogen atom or a $C_1$-$C_4$-alkyl radical or
- if $R^8$ and $R^9$ are in the ortho position relative to one another, they can together form a bridging $\alpha,\omega$-alkylenedioxo group, such as, for example, an ethylenedioxy group.

6. Composition according to Claim 5, **characterized in that** the p-phenylenediamine derivative is selected from p-phenylenediamine, p-tolylenediamine, 2-chloro-p-phenylenediamine, 2,3-dimethyl-p-phenylenediamine, 2,6-dimethyl-p-phenylenediamine, 2,6-diethyl-p-phenylenediamine, 2,5-dimethyl-p-phenylenediamine, N,N-dimethyl-p-phenylenediamine, N,N-diethyl-p-phenylenediamine, N,N-dipropyl-p-phenylenediamine, 4-amino-3-methyl-(N,N-diethyl)-aniline, N,N-bis(β-hydroxyethyl)-p-phenylenediamine, 4-N,N-bis(β-hydroxyethyl)amino-2-methylaniline, 4-N,N-bis(β-hydroxyethyl)amino-2-choroaniline, 2-(β-hydroxyethyl)-p-phenylenediamine, 2-(α,β-dihydroxyethyl)-p-phenylenediamine, 2-fluoro-p-phenylenediamine, 2-isopropyl-p-phenylenediamine, N-(β-hydroxypropyl)-p-phenylenediamine, 2-hydroxymethyl-p-phenylenediamine, N,N-dimethyl-3-methyl-p-phenylenediamine, N,N-(ethyl,β-hydroxyethyl)-p-phenylenediamine, N-(β,γ-dihydroxypropyl)-p-phenylenediamine, N-(4'-aminophenyl)-p-phenylenediamine, N-phenyl-p-phenylenediamine, 2-(β-hydroxyethyl)-p-phenylenediamine, 2-(β-acetylaminoethyloxy)-p-phenylenediamine, N-(β-methoxyethyl)-p-phenylenediamine and 5,8-diaminobenzo-1,4-dioxane and their physiologically compatible salts.

7. Composition according to Claim 6, **characterized in that** the p-phenylenediamine derivative is selected from p-phenylenediamine, p-tolylenediamine, 2-(β-hydroxyethyl)-p-phenylenediamine, 2-(α,β-dihydroxyethyl)-p-phenylenediamine and N,N-bis(β-hydroxyethyl)-p-phenylenediamine.

8. Composition according to Claim 7, **characterized in that** the p-phenylenediamine derivative is 2-(β-hydroxyethyl)-p-phenylenediamine or one of its physiologically compatible salts.

**9.** Composition according to one of Claims 1 to 8, **characterized in that** it comprises at least one coupler component.

**10.** Composition according to one of Claims 1 to 9, **characterized in that** it comprises at least one further developer component.

**11.** Composition according to one of Claims 1 to 10, **characterized in that** it further comprises at least one direct dye.

**12.** Composition according to Claim 11, **characterized in that** the direct dye is cationic.

**13.** Method for dyeing keratinous fibres, in which a composition according to one of Claims 1 to 12 is applied to the fibres and is rinsed off again after a contact time.

**Revendications**

**1.** Agent pour la teinture de fibres kératiniques, en particulier de cheveux humains contenant, dans un support acceptable du point de vue cosmétique:

(a) au moins un dérivé d'indoline ;
(b) au moins un dérivé de p-phénylènediamine ; et
(c) au moins une substance d'entretien, choisi parmi l'ectoïne ou des dérivés d'ectoïne.

**2.** Agent selon la revendication 1, **caractérisé en ce qu'**il contient un dérivé d'indoline répondant à la formule (I) ou un de ses sels physiologiquement acceptables avec un sel organique ou inorganique, dans laquelle, de manière respectivement indépendante

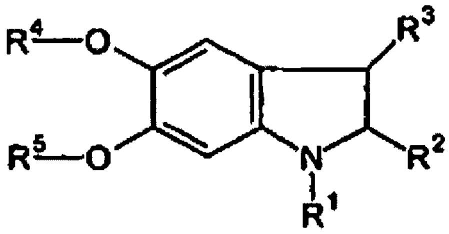

(I)

- $R^1$ représente un atome d'hydrogène, un groupe alkyle en $C_1$-$C_4$ ou un groupe hydroxyalkyle en $C_1$-$C_4$ ;
- $R^2$ représente un atome d'hydrogène ou un groupe -COOH, le groupe -COOH pouvant également être présent sous la forme d'un sel avec un cation physiologiquement acceptable ;
- $R^3$ représente un atome d'hydrogène ou un groupe alkyle en $C_1$-$C_4$;
- $R^4$ représente un atome d'hydrogène, un groupe alkyle en $C_1$-$C_4$ ou un groupe -CO-$R^6$ dans lequel $R^6$ représente un groupe alkyle en $C_1$-$C_4$ ; et
- $R^5$ représente un des groupes mentionnés sous $R^4$.

**3.** Agent selon la revendication 2, **caractérisé en ce que** le dérivé d'indoline est choisi parmi la 5,6-dihydroxyindoline, la N-méthyl-5,6-dihydroxyindoline, la N-éthyl-5,6-dihydroxyindoline, la N-propyl-5,6-dihydroxyindoline, la N-butyl-5,6-dihydroxyindoline et l'acide 5,6-dihydroxyindoline-2-carboxylique ainsi que leurs sels physiologiquement acceptables.

**4.** Agent selon la revendication 3, **caractérisé en ce que** le dérivé d'indoline est la 5,6-dihydroxyindoline ou un de ses sels physiologiquement acceptables.

**5.** Agent selon l'une quelconque des revendications 1 à 4, **caractérisé en ce qu'**il contient au moins un dérivé de p-phénylènediamine répondant à la formule (II) ou un de ses sels physiologiquement acceptables

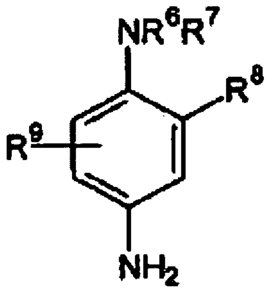

(II)

dans laquelle

$R^6$ représente un atome d'hydrogène, un résidu alkyle en $C_1$-$C_4$, un résidu monohydroxyalkyle en $C_1$-$C_4$, un résidu polyhydroxyalkyle en $C_2$-$C_4$, un résidu alcoxy(en $C_1$-$C_4$)alkyle en $C_1$-$C_4$, un résidu 4'-aminophényle ou un résidu alkyle en $C_1$-$C_4$ qui est substitué avec un groupe azoté, avec un résidu phényle ou avec un résidu 4'-aminophényle ;

$R^7$ représente un atome d'hydrogène, un résidu alkyle en $C_1$-$C_4$, un résidu monohydroxyalkyle en $C_1$-$C_4$, un résidu polyhydroxyalkyle en $C_2$-$C_4$, un résidu alcoxy(en $C_1$-$C_4$)alkyle en $C_1$-$C_4$ ou un résidu alkyle en $C_1$-$C_4$ qui est substitué avec un groupe azoté ;

$R^8$ représente un atome d'hydrogène, un atome d'halogène tel qu'un atome de chlore, un atome de brome, un atome d'iode ou un atome de fluor, un résidu alkyle en $C_1$-$C_4$, un résidu monohydroxyalkyle en $C_1$-$C_4$, un résidu polyhydroxyalkyle en $C_2$-$C_4$, un résidu hydroxyalcoxy en $C_1$-$C_4$, un résidu acétylaminoalcoxy en $C_1$-$C_4$, un résidu mésylaminoalcoxy en $C_1$-$C_4$ ou un résidu carbamoylaminoalcoxy en $C_1$-$C_4$;

$R^9$ représente un atome d'hydrogène, un atome d'halogène ou un résidu alkyle en $C_1$-$C_4$; ou bien

- lorsque les résidus $R^8$ et $R^9$ sont disposés en position ortho l'un par rapport à l'autre, ils peuvent former un groupe , $\alpha$,$\omega$-alkylènedioxo formant pont, comme par exemple un groupe éthylènedioxy.

6. Agent selon la revendication 5, **caractérisé en ce que** le dérivé de p-phénylènediamine est choisi parmi la p-phénylènediamine, la p-toluylènediamine, la 2-chloro-p-phénylènediamine, la 2,3-diméthyl-p-phénylènediamine, la 2,6-diméthyl-p-phénylènediamine, la 2,6-diéthyl-p-phénylènediamine, la 2,5-diméthyl-p-phénylènediamine, la N,N-diméthyl-p-phénylènediamine, la N,N-diéthyl-p-phénylènediamine, la N,N-dipropyl-p-phénylènediamine, la 4-amino-3-méthyl-(N,N-diéthyl)-aniline, la N,N-bis-(β-hydroxyéthyl)-p-phénylènediamine, la 4-N,N-bis-(β-hydroxyéthyl) amino-2-méthylaniline, la 4-N,N-bis-(β-hydroxyéthyl)amino-2-chloroaniline, la 2-(β-hydroxyéthyl)-p-phénylènediamine, la 2-(α,β-dihydroxyéthyl)-p-phénylènediamine, la 2-fluoro-p-phénylènediamine, la 2-isopropyl-p-phénylènediamine, la N-(β-hydroxypropyl)-p-phénylènediamine, la 2-hydroxyméthyl-p-phénylènediamine, la N,N-diméthyl-3-méthyl-p-phénylènediamine, la N,N-(éthyl-β-hydroxyéthyl)-p-phénylènediamine, la N-(β,γ-dihydroxypropyl)-p-phénylènediamine, la N-(4'-amino-phényl)-p-phénylènediamine, la N-phényl-p-phénylènediamine, la 2-(β-hydroxyéthyloxy)-p-phénylènediamine, la 2-(β-acétylaminoéthyloxy)-p-phénylènediamine, la N-(β-méthoxyéthyl)-p-phénylènediamine et le 5,8-diaminobenzo-1,4-dioxanne, ainsi que leurs sels physiologiquement acceptables.

7. Agent selon la revendication 6, **caractérisé en ce que** le dérivé de p-phénylènediamine est choisi parmi la p-phénylènediamine, la p-toluylènediamine, la 2-(β-hydroxyéthyl)-p-phénylènediamine, la 2-(α,β-dihydroxyéthyl)-p-phénylènediamine et la N,N-bis-(β-hydroxyéthyl)-p-phénylènediamine.

8. Agent selon la revendication 7, **caractérisé en ce que** le dérivé de p-phénylènediamine est la 2-(β-hydroxyéthyl)-p-phénylènediamine ou un de ses sels physiologiquement acceptables.

9. Agent selon l'une quelconque des revendications 1 à 8, **caractérisé en ce qu'**il contient au moins un composant faisant office de coupleur.

10. Agent selon l'une quelconque des revendications 1 à 9, **caractérisé en ce qu'**il contient au moins un composant supplémentaire faisant office de développeur.

11. Agent selon l'une quelconque des revendications 1 à 10, **caractérisé en ce qu'**il contient en outre au moins un colorant montant directement sur la fibre.

12. Agent selon la revendication 11, **caractérisé en ce que** le colorant montant directement sur la fibre est de type cationique.

**13.** Procédé pour la teinture de fibres kératiniques, dans lequel un agent selon l'une quelconque des revendications 1, à 12 est appliqué sur les fibres avant de l'en retirer par rinçage après l'avoir laissé agir pendant un certain temps.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 530229 B1
- WO 02096382 A
- DE 4137971
- WO 0197756 A
- EP 0671161 A1
- WO 9213829 A
- GB 1026978 A
- GB 1153196 A
- DE 2359399
- JP 02019576 A
- WO 9615765 A
- DE 3843892
- DE 4133957
- WO 9408969 A
- WO 9408970 A
- EP 740931 A
- DE 19543988
- EP 998908 A2
- DE 3725030 A
- DE 3723354 A
- DE 3926344 A

### In der Beschreibung aufgeführte Nicht-Patentliteratur

- **WALKER et al.** Test guidelines for assessment of skin compatibility of cosmetic finished products in man. *Food and Chemical Toxicology,* 1996, vol. 34, 651-660